# EUROPEAN PATENT APPLICATION

(11) **EP 4 374 803 A1**
(43) Date of publication of application: **29.05.2024**
(21) Application number: 22869307.3
(22) Date of filing: 15.09.2022
(51) Int. Cl.: A61B 17/32, A61B 18/12

(54) **ULTRASONIC SCALPEL, ENERGY INSTRUMENT FOR SURGICAL USE, AND CONTROL METHOD THEREFOR**

(30) Priority: 18.09.2021 CN 202111096716
(71) Applicant: Ensurge Medical (Suzhou) Co., Ltd., Suzhou, Jiangsu 215000 (CN)
(72) Inventor: LI, Li, Suzhou, Jiangsu 215000 (CN); WU, Zhixin, Suzhou, Jiangsu 215000 (CN)
(74) Representative: De Vries & Metman
(86) International application number: PCT/CN2022/118955
(87) International publication number: WO 2023/040939

(57) **Abstract**

An ultrasonic scalpel, an energy instrument for surgical use, and a control method therefor; the scalpel comprises a handheld component and a control module (16); the handheld component is provided with a trigger button (14); the control module (16) is disposed inside a housing of the handheld component, and the trigger button (14) is connected to an input terminal of the control module (16); the control module (16) is configured to be electrically connected to an energy generating apparatus; if the trigger button (14) is pressed, then the control module (16) controls the energy generating apparatus to output ultrasonic energy and, if the trigger button (14) is released, then the control module (16) controls the energy generating apparatus to stop outputting ultrasonic energy; and the control module (16) is configured to execute the following procedure: in response to a first trigger signal triggered by an external switching action, each time the trigger button (14) is pressed, controlling the energy generating apparatus to change the current energy output state, the changed energy output state being maintained until the trigger button (14) is pressed again. The present energy instrument for surgical use mitigates the burden and maintains the hand coordination of the surgeon.

## Description

### Technical Field

The present disclosure relates to the technical field of medical devices, and in particular, to an ultrasonic scalpel, an energy instrument for surgical use, and a control method therefor.

### Background

Ultrasonic scalpel provides ultrasonic energy in various endoscopic surgery and conventional surgery which is mainly used for hemostatic separation of soft tissue and tissue condensation. With the popularization of minimally invasive surgery, ultrasonic scalpel has become a conventional surgical instrument and is widely used. The ideal ultrasound scalpel needs to meet the following requirements simultaneously:
Firstly, it is necessary to have the safety of surgical operations. In traditional ultrasonic scalpel design, a trigger button is provided, which means that when the trigger button is pressed, the ultrasound generator is switched on to output energy, and once the trigger button is released, the ultrasound generator will be immediately switched off to stop the energy output. This can effectively reduce the risk of the ultrasound scalpel accidentally touching non surgical target human tissue in the energy excitation state, and avoid unnecessary injure, therefore, most surgeons have been trained and developed a hand-controlled mode of using trigger buttons;
Secondly, it is necessary to reduce the cost of existing ultrasound scalpels. Currently, in order to save costs, ultrasound scalpels are not used as disposable devices, so strict disinfection of the ultrasound scalpel heads is necessary to ensure sterility and safety;
Thirdly, it is necessary to have the convenience and stability of ultrasonic scalpel operation. The handheld portion of an ultrasonic scalpel usually comprises a trigger button for controlling energy output, an operation button for controlling the rotation/bending/opening and closing of the functional head, etc. When using such instrument, surgeons not only need to operate the trigger button and other different buttons, but also require different finger forces, therefore, there are high requirements for coordination between different fingers. Therefore, many doctors are accustomed to using foot switches to replace the operation that originally required one or several fingers of the surgical operator, to reduce the burden on the fingers of the surgical operator, which reduces the force and coordination requirements on the fingers of the surgical operator's hands, and makes his/her control of the instrument more stable.

The technology of ultrasonic scalpels has evolved to the point that the hand-controlled mode of trigger button in conjunction with the foot-operated mode of foot switches has become a universally accepted way of performing surgical procedures, to the point where even some doctors rely on foot switches to be able to perform their procedures at a high level.

### Summary

The present disclosure is aimed to provide an ultrasonic scalpel, an energy instrument for surgical use and a control method therefor, which replaces a foot switch while reducing the requirement for figure coordination of the surgical operator.

To achieve the above purpose, a technical solution employed by the present disclosure is:
In one aspect, the present disclosure provides an ultrasonic scalpel, the ultrasonic scalpel comprises a handheld component and a control module, the handheld component is provided with a trigger button, the trigger button is connected to an input terminal of the control module;
the control module is configured to be electrically connected to an energy generating apparatus, if the trigger button is pressed, the control module controls the energy generating apparatus to output ultrasonic energy, and if the trigger button is released, the control module controls the energy generating apparatus to stop outputting ultrasonic energy;
the control module is configured to execute the following procedure:
   in response to a first trigger signal triggered by an external switching action, each time the trigger button is pressed, the control module controls the energy generating apparatus to change the current ultrasonic energy output state, and the changed ultrasonic energy output state is maintained until the trigger button is pressed again.

Further, according to any one of the above-mentioned technical solutions and combinations of multiple technical solutions, the control module is configured to be electrically connected to the energy generating apparatus via a power board, if the trigger button is pressed, the control module controls the power board to send a drive instruction to the energy generating apparatus, and if the trigger button is released, the control module controls the power board to stop sending the drive instruction;
the control module is configured to execute the following procedure:
in response to the first trigger signal, each time the trigger button is pressed, the control module controls the power board to change the current drive instruction output state, and the changed drive instruction output state is maintained until the trigger button is pressed again, wherein, the drive instruction output state is divided into a state of sending the drive instruction and a state of stopping sending the drive instruction.

Further, according to any one of the above-mentioned technical solutions and combinations of multiple technical solutions, the control module is configured to be electrically connected to the energy generating apparatus via an electronic controllable switch, if the trigger button is pressed, the control module controls the electronic controllable switch to switch on, and if the trigger button is released, the control module controls the electronic controllable switch to switch off;
the control module is configured to execute the following procedure:
in response to the first trigger signal, each time the trigger button is pressed, the control module controls the electronic controllable switch to change the current switching state, and the changed switching state is maintained until the trigger button is pressed again.

Further, according to any one of the above-mentioned technical solutions and combinations of multiple technical solutions, the control module is further configured to execute the following procedure:
after responding to the first trigger signal, in response to a second trigger signal triggered by an external release switching action, if the trigger button is pressed, the control module controls the energy generating apparatus to output ultrasonic energy, and if the trigger button is released, the control module controls the energy generating apparatus to stop outputting ultrasonic energy;
wherein, the external release switching action is the same as or different from the external switching action.

Further, according to any one of the above-mentioned technical solutions and combinations of multiple technical solutions, the external switching action that triggers the first trigger signal comprises:
performing a predefined operation action on the trigger button, comprising double or multiple clicking the trigger button within a predefined time period.

Further, according to any one of the above-mentioned technical solutions and combinations of multiple technical solutions, the trigger button is arranged on a housing of the handheld component, there is one or more trigger buttons, and the housing of the handheld component is provided with other operation buttons thereon, and the other operation buttons comprise function keys and/or power shift buttons and/or operation buttons configured to control any of rotation, bending, opening and closing operations of a functional head.

Further, according to any one of the above-mentioned technical solutions and combinations of multiple technical solutions, the external switching action that triggers the first trigger signal comprises: performing the following operation on one or more of the trigger button and the other operation buttons:
clicking or long pressing one of the operation buttons other than the trigger button; or,
double or multiple clicking one or more of the operation buttons within a predefined time period; or,
pressing multiple operation buttons simultaneously; or,
pressing multiple operation buttons successively according to a predefined order; or,
flipping a dial switch in the operation buttons.

Further, according to any one of the above-mentioned technical solutions and combinations of multiple technical solutions, no joint or interface is provided on the handheld component to connect to a foot switch.

Further, according to any one of the above-mentioned technical solutions and combinations of multiple technical solutions, the handheld component comprises a handle shell, a transducer assembly and a power cord, wherein, the control module and the transducer assembly are arranged within an accommodating cavity of the handle shell, and the transducer assembly is rotatably arranged around an axis in the accommodating cavity;
one end portion of the power cord is connected to the transducer assembly in the accommodating cavity, and the other end portion of the power cord goes through a lower portion of the handle shell to the outside of the accommodating cavity.

Further, according to any one of the above-mentioned technical solutions and combinations of multiple technical solutions, the transducer assembly comprises a transducer housing and a transducer that are fixedly arranged to each other, the transducer housing has a hollow cavity, at least a rear portion of the transducer is accommodated within the hollow cavity, and the transducer assembly further comprises a conductive element fixedly arranged outside the transducer housing;
the conductive element has at least a conductive portion, the transducer is fixedly and electrically connected to the conductive portion, the accommodating cavity of the handle shell is further fixedly provided with a power connection element therein, the power connection element abuts against the conductive portion, and in the process that the transducer assembly rotates about its own axis with respect to the handle shell, the power connection element is always in contact with the conductive portion to maintain electric connection.

Further, according to any one of the above-mentioned technical solutions and combinations of multiple technical solutions, the conductive element is fixedly arranged at an outer peripheral portion of the transducer housing, the conductive portion is in a shape of a circular ring, and the axis of the conductive portion is collinear with the axis of the transducer assembly, the power connection element is located on the circumferential outer side of the transducer assembly, and the power connection element elastically abuts against the conductive portion inwards along the radial direction of the transducer housing.

Further, according to any one of the above-mentioned technical solutions and combinations of multiple technical solutions, the conductive element is fixedly arranged at a rear end portion of the transducer housing, and the power connection element elastically abuts against the conductive portion forwards along a front-rear direction of the transducer housing.

Further, according to any one of the above-mentioned technical solutions and combinations of multiple technical solutions, the ultrasonic scalpel further comprises a cutting tool, the cutting tool comprises an inner tube, an outer tube and a tool bar which extend in the front-rear direction, wherein the tool bar runs through the inner tube, the outer tube is sleeved outside the inner tube, the tool bar and the outer tube are fixed to each other and are arranged in a manner that can move synchronously with respect to the inner tube forward and backward, wherein a rear portion of the tool bar is fixedly connected to the transducer assembly.

Further, according to any one of the above-mentioned technical solutions and combinations of multiple technical solutions, in response to the first trigger signal, the external switching action that triggers the first trigger signal comprises:
the control module is connected to an external human-machine interaction device through a wired or wireless mode, and an instruction is input to the control module through the human-machine interaction device.

Further, according to any one of the above-mentioned technical solutions and combinations of multiple technical solutions, the human-machine interaction device is a touch screen, and the touch screen is in wired connection to the control module; or, the human-machine interaction device is a mobile terminal, and the mobile terminal is wirelessly connected to the control module.

In another aspect, the present disclosure provides an energy instrument for surgical use having a first working mode and a second working mode, the energy instrument for surgical use comprises:
an energy generating apparatus configured to generate energy;
a handheld component electrically connected to the energy generating apparatus via an electronic controllable switch, the handheld component being provided with one or more operation buttons, wherein at least one of the operation buttons is a trigger button;
a control module with an input terminal electrically connected to the operation buttons, and an output terminal electrically connected to the electronic controllable switch; the control module being configured to control the energy instrument for surgical use to switch from the first working mode to the second working mode according to a first trigger signal triggered by an external switching action, and to switch from the second working mode to the first working mode according to a second trigger signal triggered by an external release switching action, the external release switching action being the same as or different from the external switching action;
when the energy instrument for surgical use is in the first working mode, if the trigger button is pressed, the control module controls the energy generating apparatus to output ultrasonic energy, and if the trigger button is released, the control module controls the energy generating apparatus to stop outputting ultrasonic energy;
when the energy instrument for surgical use is in the second working mode, in response to that each time the trigger button is pressed, the control module controls the energy generating apparatus to change the current energy output state, and the changed energy output state is maintained until the trigger button is pressed again.

Further, according to any one of the above-mentioned technical solutions and combinations of multiple technical solutions, the control module is configured to be electrically connected to the energy generating apparatus via a power board, if the trigger button is pressed, the control module controls the power board to send a drive instruction to the energy generating apparatus, and if the trigger button is released, the control module controls the power board to stop sending the drive instruction;
the control module is configured to execute the following procedure:
in response to the first trigger signal, each time the trigger button is pressed, the control module controls the power board to change the current drive instruction output state, and the changed drive instruction output state is maintained until the trigger button is pressed again, wherein, the drive instruction output state is divided into a state of sending the drive instruction and a state of stopping sending the drive instruction.

Further, according to any one of the above-mentioned technical solutions and combinations of multiple technical solutions, the control module is configured to be electrically connected to the energy generating apparatus via an electronic controllable switch, if the trigger button is pressed, the control module controls the electronic controllable switch to switch on, and if the trigger button is released, the control module controls the electronic controllable switch to switch off;
the control module is configured to execute the following procedure:
in response to the first trigger signal, each time the trigger button is pressed, the control module controls the electronic controllable switch to change the current switching state, and the changed switching state is maintained until the trigger button is pressed again.

Further, according to any one of the above-mentioned technical solutions and combinations of multiple technical solutions, the control module controlling the instrument to switch the working mode according to the first trigger signal and the second trigger signal comprises:
one or more of the operation buttons are configured to act in accordance with a predefined operation to trigger the control module.

Further, according to any one of the above-mentioned technical solutions and combinations of multiple technical solutions, one or more of the operation buttons are configured to trigger the control module according to the following operation mode:
clicking or long pressing one of the operation buttons other than the trigger button; or,
double or multiple clicking one or more of the operation buttons; or,
pressing multiple operation buttons simultaneously; or,
pressing multiple operation buttons successively according to a predefined order; or,
flipping a dial switch in the operation buttons.

Further, according to any one of the above-mentioned technical solutions and combinations of multiple technical solutions, the operation buttons comprise a trigger button and other operation buttons, and the other operation buttons comprise function keys and/or power shift buttons and/or operation buttons configured to control any of rotation, bending, opening and closing operations of a functional head.

Further, according to any one of the above-mentioned technical solutions and combinations of multiple technical solutions, the control module controlling the instrument to switch the working mode according to the first trigger signal and the second trigger signal comprises:
the control module is connected to an external human-machine interaction device through a wired or wireless mode, and an instruction is input to the control module through the human-machine interaction device to trigger the control module.

Further, according to any one of the above-mentioned technical solutions and combinations of multiple technical solutions, the human-machine interaction device is a touch screen, and the touch screen is in wired connection to the control module; or,
the human-machine interaction device is a mobile terminal, and the mobile terminal is wirelessly connected to the control module.

Optionally, after being powered on, the energy instrument for surgical use is initialized to the first working mode, or
after being powered on, the energy instrument for surgical use is initialized to the second working mode.

Optionally, the energy instrument for surgical use is an ultrasonic scalpel, the energy generating apparatus is an ultrasonic generator, and the ultrasonic generator is arranged inside or outside the handheld component; or,
the energy instrument for surgical use is a laser knife, the energy generating apparatus is a laser generator, and the laser generator is arranged inside or outside the handheld component; or,
the energy instrument for surgical use is an electric knife, the energy generating apparatus is an electrical signal generator, and the electrical signal generator is arranged inside or outside the handheld component; or,
the energy instrument for surgical use is a medical smoking device, and the energy generating apparatus is an air pump; or,
the energy instrument for surgical use is a vacuum suction apparatus, and the energy generating apparatus is a vacuum pump; or,
the energy instrument for surgical use is an infusion pump, and the energy generating apparatus is an infusion pump; or,
the energy instrument for surgical use is a manipulator with an elbow joint rotation, and the energy generating apparatus is a motor; or,
the energy instrument for surgical use is a monopolar electrocoagulation pencil or bipolar electrocoagulation forceps, and the energy generating apparatus is an electrical signal generator.

In a third aspect, the present disclosure provides a control method for an energy instrument for surgical use, the controlled energy instrument for surgical use comprises a trigger button, an initial control mode of the trigger button on energy output is: if the trigger button is pressed, the instrument outputs energy, and if the trigger button is released, the instrument stops outputting energy, which changes the control mode of the trigger button on energy output without a foot switch, and the control method comprises the following steps:
in the case that the energy instrument for surgical use is powered on, inputting a predefined trigger action to redefine a control mode of the trigger button on energy output to: no energy output in an initial state, and if the trigger button is pressed for the first time, the instrument outputs energy, and the instrument will not stop outputting energy until the trigger button is pressed again;
the trigger button will not restore the initial control mode on energy output until the energy instrument for surgical use is disconnected from the power supply or a predefined trigger action is input.

Further, the control mode is applied to the following energy instrument for surgical use:
the energy instrument for surgical use is an ultrasonic scalpel, the energy generating apparatus is an ultrasonic generator, and the ultrasonic generator is arranged inside or outside the handheld component; or,
the energy instrument for surgical use is a laser knife, the energy generating apparatus is a laser generator, and the laser generator is arranged inside or outside the handheld component; or,
the energy instrument for surgical use is an electric knife, the energy generating apparatus is an electrical signal generator, and the electrical signal generator is arranged inside or outside the handheld component; or,
the energy instrument for surgical use is a medical smoking device, and the energy generating apparatus is an air pump; or,
the energy instrument for surgical use is a vacuum suction apparatus, and the energy generating apparatus is a vacuum pump; or,
the energy instrument for surgical use is an infusion pump, and the energy generating apparatus is an infusion pump; or,
the energy instrument for surgical use is a manipulator with an elbow joint rotation, and the energy generating apparatus is a motor; or,
the energy instrument for surgical use is a monopolar electrocoagulation pencil or bipolar electrocoagulation forceps, and the energy generating apparatus is an electrical signal generator.

The beneficial effects brought by the technical solutions provided by the present disclosure are as follows:
a. The hand-controlled mode of the trigger button of the traditional ultrasonic scalpel is reserved such that starting and stopping of energy excitation can be precisely controlled even in regions with smaller areas in a surgical target human tissue, improving the surgical safety and reducing unnecessary surgical trauma;
b. An energy instrument for surgical use with different working modes that can be flexibly switched is provided, where the working modes can be switched during surgical stage where the surgeon can control the safety of the operation (such as when the surgical target human tissue has a large area), that is, the energy output can be maintained continuously without pressing the trigger button, which is beneficial for improving the smooth operation level of the instrument and reducing the burden on the surgeon's fingers;
c. It can meet the requirements of reducing the force and coordination of the surgical operator's hands without using the foot switch.

### Brief Description of the Drawings

For more clearly explaining the technical solutions in the embodiments of the present application or in the prior art, the accompanying drawings required to be used to in the description of the embodiments or the prior art will be simply introduced below. Apparently, the drawings in the following description show merely some embodiments described in the present application, and those of ordinary skill in the art may derive other drawings from these accompanying drawings without creative efforts.
Figure 1 is a schematic structure diagram of an ultrasonic scalpel provided by an exemplary embodiment of the present disclosure;
Figure 2 is a schematic overall structure diagram of an ultrasonic scalpel handle provided by an exemplary embodiment of the present disclosure;
Figure 3 is a schematic exploded structure diagram of an ultrasonic scalpel handle provided by an exemplary embodiment of the present disclosure;
Figure 4 is a schematic internal structure diagram of an ultrasonic scalpel handle provided by an exemplary embodiment of the present disclosure;
Figure 5 is a schematic diagram of the connection between the transducer assembly and the power connection element in an ultrasonic scalpel handle provided by an exemplary embodiment of the present disclosure;
Figure 6 is a schematic overall structure diagram of a transducer assembly provided by an exemplary embodiment of the present disclosure;
Figure 7 is a schematic exploded structure diagram of the transducer assembly of Figure 6;
Figure 8 is a front view of the transducer assembly of Figure 6;
Figure 9 is a schematic sectional view along Line A1-A1 in Figure 8;
Figure 10 is a schematic enlarged diagram at Part D in Figure 8;
Figure 11 is a schematic overall structure diagram of an ultrasonic scalpel handle provided by another exemplary embodiment of the present disclosure;
Figure 12 is a schematic exploded structure diagram of an ultrasonic scalpel handle provided by another exemplary embodiment of the present disclosure;
Figure 13 is a schematic overall structure diagram of a transducer assembly provided by another exemplary embodiment of the present disclosure;
Figure 14 is a schematic exploded structure diagram of the transducer assembly of Figure 13;
Figure 15 is a front view of the transducer assembly of Figure 13;
Figure 16 is a schematic sectional view along Line A2-A2 in Figure 15;
Figure 17 is a left view of the transducer assembly of Figure 13;
Figure 18 is a schematic structure diagram of the connection between the transducer assembly and the power connection element provided by another exemplary embodiment of the present disclosure;
Figure 19 is a schematic internal structure diagram of an ultrasonic scalpel handle provided by an exemplary embodiment of the present disclosure;
Figure 20 is a schematic diagram of an operation button of a handheld component provided by an exemplary embodiment of the present disclosure;
Figure 21 is a schematic diagram of the working mode switching of an energy instrument for surgical use provided by an exemplary embodiment of the present disclosure.

Wherein, the references comprise: 1, ultrasonic scalpel handle; 11, handle shell; 11a, left shell; 11b, right shell; 11c, top cover; 11d, perspective window; 12, transducer assembly; 121, transducer housing; 1211, front housing; 1212, rear housing; 1213, middle housing; 121a, straight ring gear; 122, transducer; 1221, horn shaft; 1222, horn core; 1223, retaining ring; 1224, first power connection wire/ second power connection wire; 123, conductive element; 1231, first conductive ring (first conductive portion); 1232, second conductive ring (second conductive portion); 1233, spacer ring; 1234, insulation sleeve; 1235, positioning lug boss; 1230, plate body; 123a, first conductive piece; 123b, second conductive piece; 123c, first perforated hole; 123d, second perforated hole; 124, connecting screw; 125, rubber gasket; 126, rubber ring; 127, front retaining cover; 128, rear sealing ring; 129, screw; 13, power connection element; 131, first power connection element; 132, second power connection element; 13a, first elastic power connection piece; 13b, second elastic power connection piece; 14, trigger button; 15, other operation button; 16, control module; 2, cutting tool; 3, power adapter; 31, connecting wire; 30, connecting wire tip.

### Detailed Description of Exemplary Embodiments

In order to make those skilled in the art better understand the solutions of the present disclosure, the technical solutions in the embodiments of the present disclosure will be described clearly and completely below with reference to the accompanying drawings in the embodiments of the present disclosure. Apparently, the described embodiments are merely some of rather than all of the embodiments of the present disclosure. All other embodiments obtained by those of ordinary skill in the art without creative efforts based on the embodiments of the present disclosure shall fall within the protective scope of the present disclosure.

It should be noted that terms such as "first" and "second" in the description, the claims and the accompanying drawings of the present disclosure are used to/intended to distinguish similar objects, and do not have to be used to/are not intended to describe a specific order or sequence. It should be understood that data used in this manner may be interchangeable where appropriate, so that the embodiments of the present disclosure described herein can be implemented in an order other than/in addition to those illustrated or described herein. In addition, terms "comprise" and "have" and any other variants thereof are intended to cover non-exclusive inclusion, for example, a process, method, device, product, or equipment that includes a series of steps or units is not necessarily limited to those clearly listed steps or units, but may include other steps or units not listed clearly or inherent to the process, method, product, or equipment.

As stated in the Background, many energy instruments for surgical use provide foot switches for surgical operators to select and use, to mitigate the finger burden and reduce finger coordination requirements. However, the foot switches bring at least the following defects:
First, due to that a doctor only needs one foot to control the foot switch, most of the weight of the body needs to be borne by the other leg, considering that some surgeries take a long time, with some lasting more than ten hours, the frequent surgical work throughout the year makes the load-bearing leg (the other leg other than the leg stepping on the foot switches) of the doctor who is accustomed to using foot switches very pone to fatigue and even damage (including muscles, joints, varicose veins, etc.);
Second, not every surgeon is accustomed to using foot switches, but as long as a hospital has one surgeon who needs to use them, the hospital needs to purchase them, the market price of a foot switch is even more expensive than an energy instrument for surgical use, leading to a significant increase in the unit cost of procurement, and operating room equipment needs to be regularly disinfected and maintained, using foot switches will increase the management costs;
Third, a plurality of instruments may be controlled by foot switches during surgery, which may result in the surgeons having more than one foot switch under their feet, when there are too many things on the ground, especially during surgery when the surgeons and nurses are highly focused on the surgery and neglect to observe the ground, they are prone to tripping when walking;
Fourth, if there are multiple surgical procedures where different surgeons have foot switch needs, and in case that it needs to take turns to use the foot switch, if the surgical schedule makes the needs of the foot switch occur at the same time and conflict with each other, it can seriously affect the qualities of surgeries or disrupt existing surgical schedules.

However, due to the fact that the combination of the scalpels and the foot switches has become an inherent mindset, it is not easy to think of changing this pattern habit that has been used for decades, even many experienced surgeons cannot perform surgeries smoothly without foot switches, therefore, even though poor standing posture during prolonged surgeries throughout the year puts a hug burden on their legs, they still cannot give up the using of foot switches.

In one embodiment of the present disclosure, an ultrasonic scalpel is provided, which comprises a handheld component and a control module, the handheld component is provided with a trigger button, the trigger button is connected to an input terminal of the control module;

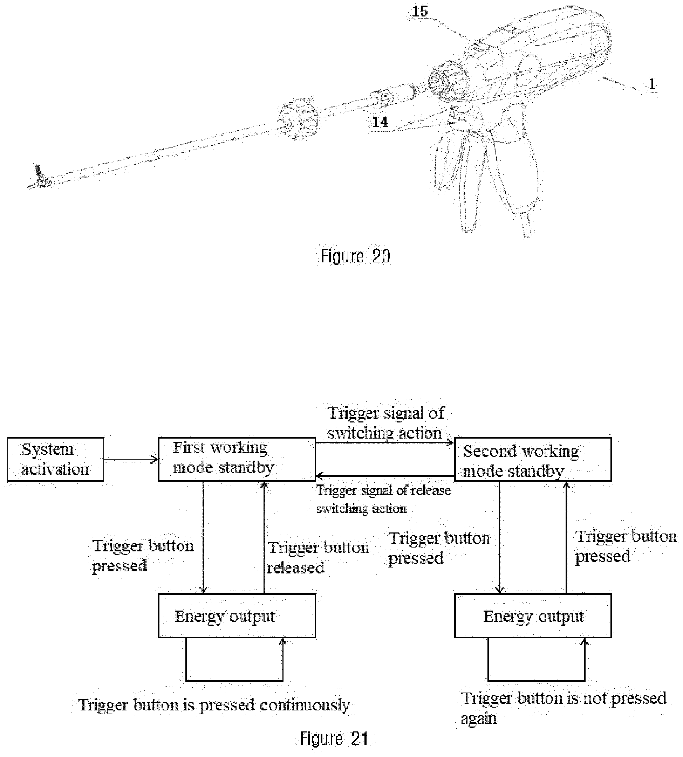

The output terminal of the control module is electrically connected to the energy generating apparatus directly or indirectly, in this embodiment, the two is connected via an electronic controllable switch, or may be connected directly via a wire in other embodiments, in the case of being connected via an electronic controllable switch, if the trigger button is pressed, the control module controls the electronic controllable switch to switch on, and if the trigger button is released, the control module controls the electronic controllable switch to switch off;
Referring to Figure 21, the control module is configured to execute the following procedure:
in response to a first trigger signal triggered by an external switching action, the control module switches the working mode of the trigger button to: each time the trigger button is pressed, the control module controls the electronic controllable switch to change the current switching state, and the changed switching state is maintained until the trigger button is pressed again, for example: on the premise of switching to this working mode, the energy instrument for surgical use is currently in standby mode (the electronic controllable switch is off). If the trigger button is pressed, the control module controls the electronic controllable switch to switch on, at this moment the operating system of the energy instrument for surgical use closes the circuit, the energy generating apparatus continuously outputs energy to the handheld component, even if the trigger button is released, the energy output is maintained continuously; the control module will not control the electronic controllable switch to switch off until the trigger button is pressed again, at this moment the operating system cuts off the circuit, at this moment the energy generating apparatus stops outputting energy to the handheld component, and returns to the standby mode.

In another embodiment of the present disclosure, the control module is electrically connected to the energy generating apparatus directly via a wire rather than an electronic controllable switch, that is, the control module may directly drive the energy generating apparatus, if the trigger button is pressed, it drives the energy generating apparatus to output energy; if the trigger button is released, the energy generating apparatus will not be driven, and accordingly, the energy generating apparatus will stop outputting energy. The control module is configured to execute the following procedure: in response to a first trigger signal triggered by an external switching action, each time the trigger button is pressed, the control module controls the energy generating apparatus to change the current ultrasonic energy output state, and the changed ultrasonic energy output state is maintained until the trigger button is pressed again, for example, if the energy generating apparatus is currently not outputting energy, and if the trigger button is pressed at this moment, even if the trigger button is released at this moment, the energy generating apparatus will continue to output energy, and the energy generating apparatus will not stop outputting energy until the trigger button is pressed again; if the energy generating apparatus is expected to continuously output energy again, it only needs to press the trigger button again, and repeat the process.

In another embodiment of the present disclosure, the control module is electrically connected to the energy generating apparatus via a power board rather than the electronic controllable switch, if the trigger button is pressed, the control module controls the power board to send a drive instruction to the energy generating apparatus, and if the trigger button is released, the control module controls the power board to stop sending the drive instruction; the control module is configured to execute the following procedure: in response to the first trigger signal, each time the trigger button is pressed, the control module controls the power board to change the current drive instruction output state, and the changed drive instruction output state is maintained until the trigger button is pressed again, wherein, the drive instruction output state is divided into a state of sending the drive instruction and a state of stopping sending the drive instruction, for example, if the energy generating apparatus is currently not outputting energy, and if the trigger button is pressed at this moment, even if the trigger button is released at this moment, the power board will still continue to send the drive instruction to the energy generating apparatus, to cause it to continue to output energy, and the power board will not stop sending the drive instruction to the energy generating apparatus, and the energy generating apparatus will not stop outputting energy accordingly until the trigger button is pressed again; if the energy generating apparatus is expected to continuously output energy again, it only needs to press the trigger button again, and repeat the process.

The handheld component of this embodiment can achieve the "foot mode" without being connected to a foot switch, the "foot mode" herein does not refer to the mode of controlling the operation of the energy generating apparatus by stepping on a button on a foot switch, but rather to the traditional working logic (working mode) of changing the trigger button through the first trigger signal or a second trigger signal triggered by an external release switching action, so that users can continuously excite energy without having to keep pressing the trigger button with their fingers all the time, as if stepping on the foot switch.

To facilitate the description of the relative positions of the components in the ultrasonic scalpel, the above and following descriptions of the front-rear direction are defined with reference to the direction observed by the operator while holding the ultrasonic scalpel for operation, where the position of the ultrasonic scalpel acting on the surgical site is front, and the position of the ultrasonic scalpel near the body of an operator (doctor) is rear.

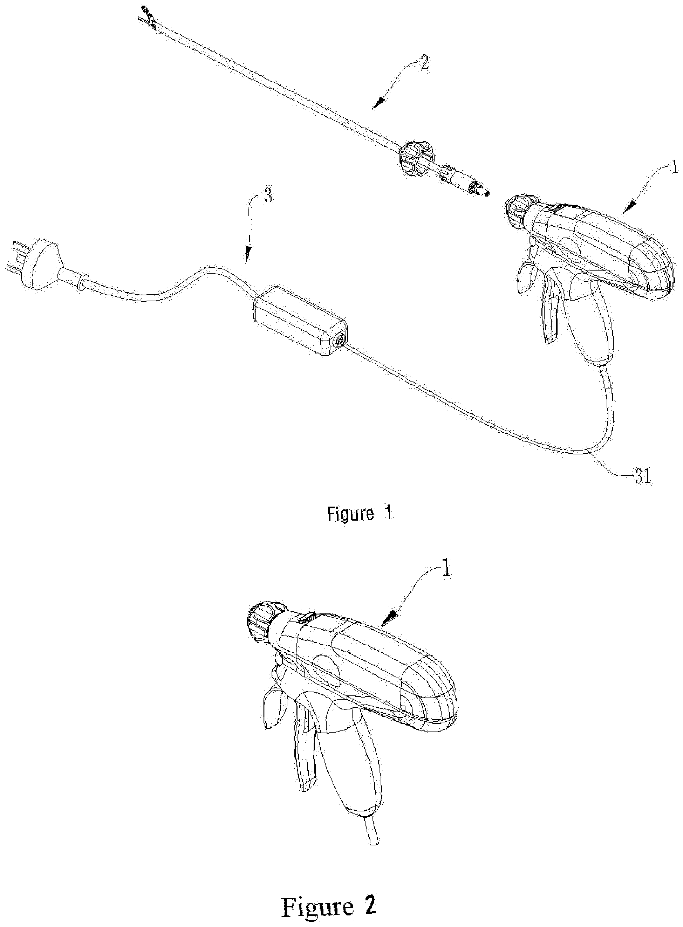

Referring to the ultrasonic scalpel system shown in Figure 1, it comprises an ultrasonic scalpel, and a power adapter 3 for supplying energy to the ultrasonic scalpel; wherein the ultrasonic scalpel comprises an ultrasonic scalpel handle 1, and a cutting tool 2 detachably mounted on the ultrasonic scalpel handle 1.

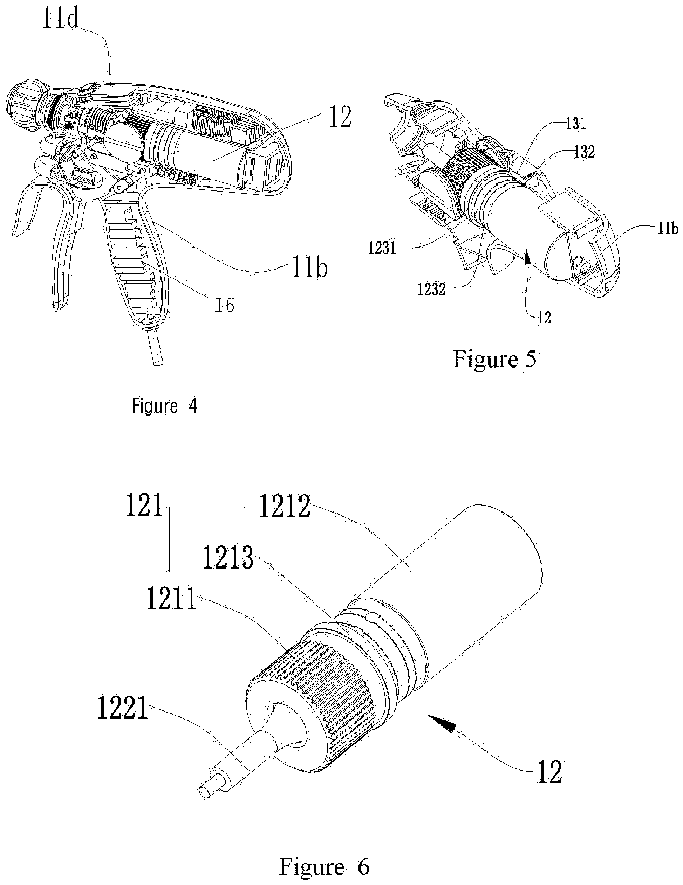

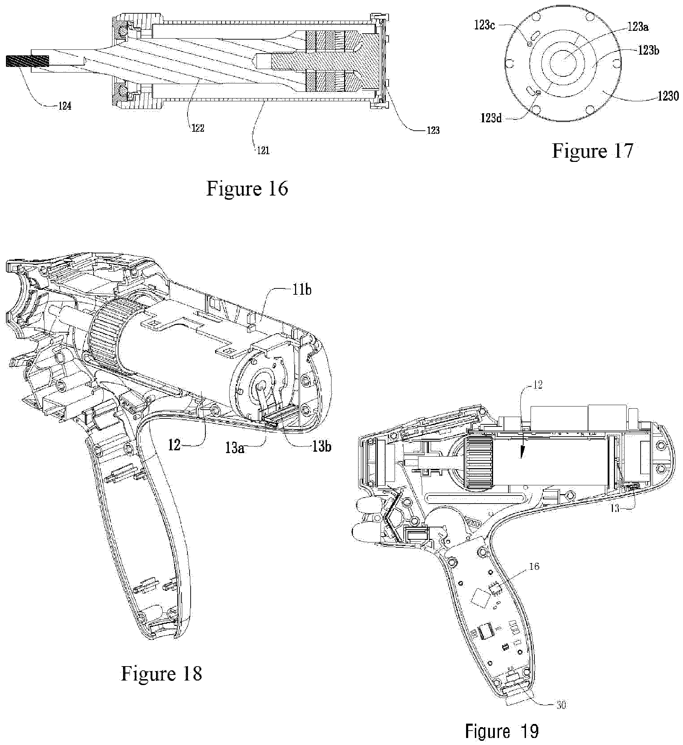

Referring to the drawings, the ultrasonic scalpel handle 1 comprises a handle shell 11 and a transducer assembly 12, the handle shell 11 comprises a left shell 11a and a right shell 11b that are fixedly connected and fitted, and a top cover 11c located at the top, the handle shell 11 has an accommodating cavity, the transducer assembly 12 is integrally accommodated in the accommodating cavity and can be rotatably arranged about its own axis, and the control module 16 is optionally arranged in the accommodating cavity, as shown in Figure 4 and Figure 19. The transducer assembly 12 comprises a transducer housing 121 and a transducer 122 that are fixedly to each other, the transducer housing 121 has a hollow cavity, and the transducer 122 is accommodated in the above hollow cavity at least at the rear portion thereof and is fixed with respect to the transducer housing 121.

Specifically, referring to the figures, the transducer 122 is an integral element, and comprises a horn shaft 1221 and a horn core 1222 successively arranged along an axial direction, a retaining ring 1223 is formed at the position where the horn shaft 1221 is connected to the horn core 1222, the horn core 1222 and the retaining ring 1223 are all accommodated in the hollow cavity of the transducer housing 121, and the front portion of the horn shaft 1221 extends out of the hollow cavity and is connected to the cutting tool 2 through the front connecting screw 124.

The transducer assembly 12 further comprises a conductive element 123 fixedly arranged on the outer side of the transducer housing 121, the conductive element 123 has at least a conductive portion, the transducer 122 is fixedly and electrically connected to the conductive portion mentioned above, the accommodating cavity of the handle shell 11 is further fixedly provided with a power connection element 13 therein, the power connection element 13 abuts against the conductive portion, and in the process that the transducer 122 rotates about its own axis with respect to the handle shell 11, the power connection element 13 is always in contact with the conductive portion to maintain electric connection.

In a structural embodiment of an ultrasonic scalpel of the present disclosure, as shown in Figure 2 to Figure 10, the conductive element 123 is fixedly arranged at an outer peripheral portion of the transducer housing 121, the conductive portion is in a shape of a circular ring, and the axis of the conductive portion extends collinear with the axis of the transducer assembly 12. Specifically, the conductive portion comprises a first conductive portion and a second conductive portion that are insulated from each other and are both in a shape of a circular ring, the transducer 122 has two power connection wires 1224 a first power connection wire and a second power connection wire, and the two power connection wires are electrically connected to the two conductive portions, respectively.

The two conductive portions are arranged at intervals along the axial direction of the transducer housing 121, in this embodiment, the conductive element 123 comprises two conductive rings both made of a conductive material a first conductive ring 1231 and a second conductive ring 1232, and a spacer ring 1233 arranged between the first conductive ring 1231 and the second conductive ring 1232 and made of an insulation material, the first conductive ring 1231 forms the first conductive portion and the second conductive ring 1232 forms the second conductive portion. An end portion of the first power connection wire 1224 is fixedly arranged on the first conductive ring 1231 to realize electric connection, and an end portion of the second power connection wire 1224 is fixedly arranged on the second conductive ring 1232 to realize electric connection.

The first conductive ring 1231, the second conductive ring 1232 and the spacer ring 1233 are relatively fixedly sleeved on the outer peripheral portion of the transducer housing 121, specifically, a circumferential limit structure is provided between each conductive ring and the spacer ring 1233 to limit their relative rotation, and a positioning structure is further provided between the entire conductive element 123 and the transducer housing 121 to limit the rotation of the conductive element 123 and the axial movement of the conductive element 123.

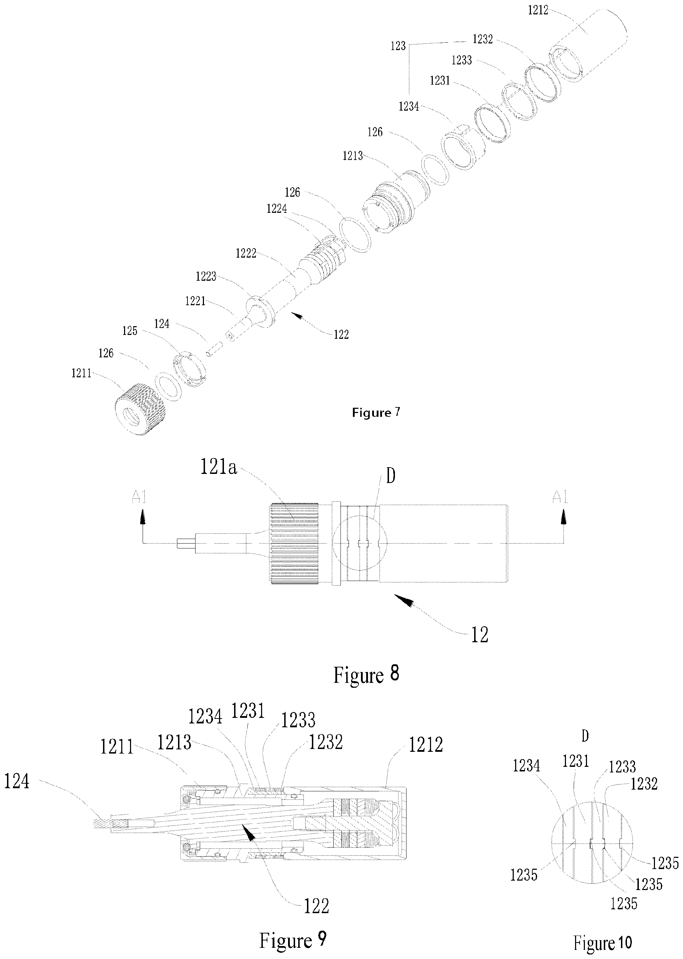

Here, as shown in Figure 6 and Figure 7, the transducer housing 121 comprises a front housing 1211, a middle housing 1213 and a rear housing 1212 successively arranged in a front-rear direction, a rubber gasket 125 and a rubber ring 126 are provided between the front housing 1211 and the middle housing 1213 to realize sealing, a rubber ring 126 is provided between the middle housing 1213 and the rear housing 1212 to realize sealing, and meanwhile, the transducer 122 accommodated in the transducer housing 121 is sealed in the transducer housing 121. The conductive element 123 is fixedly mounted on the housing 1213 and is located at the front portion of the rear housing 1212.

The conductive element 123 further comprises an insulation sleeve 1234, the insulation sleeve 1234 is fixedly sleeved on an outer peripheral portion of the middle housing 1213, the first conductive ring 1231, the spacer ring 1233 and the second conductive ring 1232 are together sleeved on an outer peripheral portion of a rear segment of the insulation sleeve 1234, and the front portion of the first conductive ring 1231 and the insulation sleeve 1234, the rear portion of the first conductive ring 1231 and the front portion of the spacer ring 1233, the rear portion of the spacer ring 1233 and the front portion of the second conductive ring 1232, and the rear portion of the second conductive ring 1232 and the front portion of the rear housing 1212 are all located in cooperation with each other by means of positioning lug bosses 1235 and recesses, in this way, the conductive element 123 is fixed on the transducer housing 121.

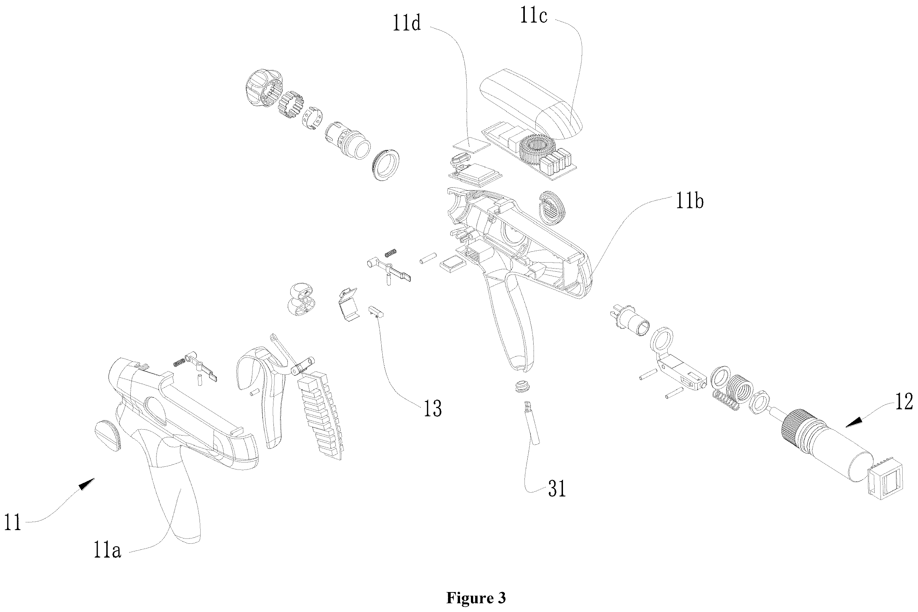

As shown in Figure 3 and Figure 5, the handle shell 11 is provided with a power connection element 13, the power connection element 13 is located on the circumferential outer side of the transducer assembly 12, the power connection element 13 is against the outer peripheral portion of the transducer assembly 12 and abuts against the conductive portion, specifically, the power connection element 13 comprises a first power connection element 131 and a second power connection element 132 that are independent of each other, the first power connection element 131 and the second power connection element 132 are both made of metal material and both have a certain degree of elasticity along their own length direction, and are arranged at intervals along the front-rear direction of the handle shell 11, the first power connection element 131 elastically abuts against the outer peripheral portion of the first conductive ring 1231 radially inwards along the transducer housing 121, and the second power connection element 132 elastically abuts against the outer peripheral portion of the second conductive ring 1232 radially inwards along the transducer housing 121. During the rotation process of the transducer assembly 12 in its entirety around its own axis with respect to the handle shell 11, the first power connection element 131 and the first conductive ring 1231, and the second power connection element 132 and the second conductive ring 1232 are contact with each other all the time to maintain electric connection.

Referring to Figure 5, in this embodiment, the power connection element 13 is arranged in the handle shell 11 and located at an upper position, and is located above the transducer assembly 12, such that the power connection element 13 can abut against the outer peripheral portion of the transducer assembly 12 downward to maintain electric connection more stably.

In this way, it only needs to connect the power cord to the power connection element 13 inside the handle shell 11, then the power cord can be led from the lower portion of the handle shell 11 to connect to the power supply, in this way, during the rotation process of the transducer assembly 12 about its own axis in the handle shell 11, the power cord does not rotate along with it, such that a series of problems of the large arm force and easy fatigue, and knotting of the power cord, etc. caused by the power cord extending from the rear portion of the handle shell 11 are avoided.

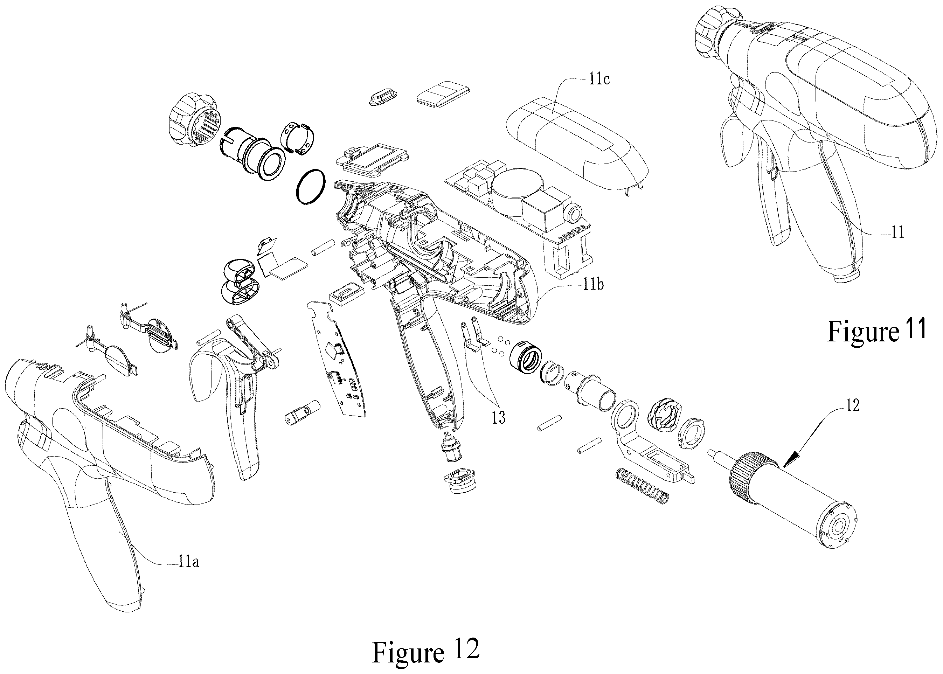

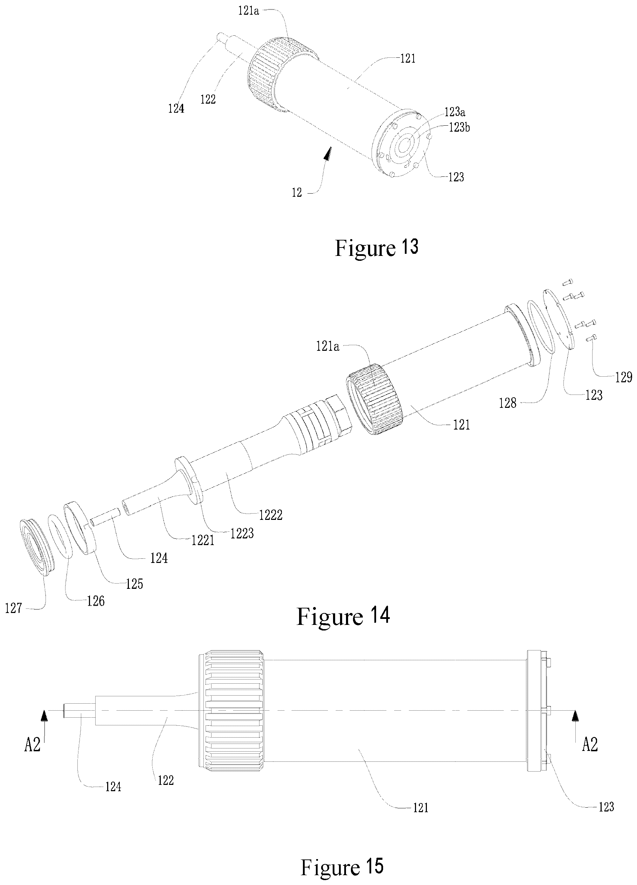

In a structural embodiment of an ultrasonic scalpel of the present disclosure, as shown in Figure 11 to Figure 19, in this embodiment, the conductive element 123 is fixedly arranged at a rear end portion of the transducer housing 121, the transducer housing 121 can be provided open at its rear portion, and the conductive element 123 seals the transducer housing 121 from the rear end. The conductive portions are arranged at the rear portion of the conductive element 123, the power connection element 13 is arranged in the handle shell 11 and located behind the transducer assembly 12, and the power connection element 13 elastically abuts against the conductive portion forward.

Specifically, the conductive portion comprises a first conductive portion and a second conductive portion insulated from each other, the transducer 122 has two power connection wires a first power connection wire and a second power connection wire (not shown), the first power connection wire is electrically connected to the first conductive portion, and the second power connection wire is electrically connected to the second conductive portion. The power connection element 13 also comprises a first elastic power connection piece 13a and a second elastic power connection piece 13b disposed independently and insulated from each other, the first elastic power connection piece 13a abuts against the first conductive portion, and the second elastic power connection piece 13b abuts against the second conductive portion. The above first conductive portion and the second conductive portion are in the shape of a disc or circle taking the axis line of the transducer assembly 12 as a rotation center, in this way, in the process that the transducer assembly 12 rotates about its own axis, the first elastic power connection piece 13a and the second elastic power connection piece 13b can keep to abut against the first conductive portion and the second conductive portion respectively.

In this embodiment, the conductive element 123 comprises a plate body 1230, and a first conductive piece 123a and a second conductive piece 123b which are fixed on the plate body 1230 and made of metal materials, wherein, the first conductive piece 123a is in the shape of a disc, and the second conductive piece 123b is in the shape of a circle and circumferentially disposed on the circumferential outer side of the first conductive piece 123a, and the first conductive piece 123a and the second conductive piece 123b are disposed at intervals along the radial direction of the conductive element 123, that is, the outer circumferential wall of the first conductive piece 123a and the inner circumferential wall of the second conductive piece 123b have a distance in the radial direction of the conductive element 123. The first conductive piece 123a forms the first conductive portion, and the second conductive piece 123b forms the second conductive portion.

A first perforated hole 123c and a second perforated hole 123d are opened on the plate body 1230 penetrating in its own thickness direction, the first power connection wire runs through the first perforated hole 123c and fixedly to the first conductive piece 123a by means of welding to realize electric connection, and the second power connection wire runs through the second perforated hole 123d and fixedly to the second conductive piece 123b by means of welding to realize electric connection.

Referring to Figure 12 and Figure 18, the first elastic power connection piece 13a and the second elastic power connection piece 13b are both elastic pieces made of metal materials, the lower end portion of the first elastic power connection piece 13a and the lower end portion of the second elastic power connection piece 13b are fixedly disposed in the handle shell 11 respectively, the upper end portion of the first elastic power connection piece 13a presses against the rear side of the first conductive portion 123 a forward, and the upper end portion of the second elastic power connection piece 13b presses against the rear side of the second conductive portion 123b forward.

In this way, it only needs to connect the two conductive wires of the power cord to the first elastic power connection piece 13a and the second elastic power connection piece 13b respectively, then the power cord can be led from the lower portion of the handle shell 11 to connect to the power supply, in this way, in the rotation process of the transducer assembly 12 about its own axis in the handle shell 11, the power cord does not rotate along with it, such that a series of problems of the large arm force and easy fatigue, and knotting of the power cord, etc. caused by the power cord extending from the rear portion of the handle shell 11 are avoided.

The ultrasonic scalpel of this embodiment may also be switched back to its previous working mode through a second trigger signal triggered by an external release switching action, specifically, the control module is further configured to execute the following procedure:
in response to the second trigger signal, the control module restores the working mode of the trigger button to the working mode before switching; wherein, the external release switching action is the same as or different from the external switching action.

The specific operation of switching the working mode according to the first trigger signal and the second trigger signal mentioned above is as follows (as the switching principle of the first trigger signal and the second trigger signal is the same, the following only takes the external switching action used to trigger the first trigger signal as an example for explanation):
The first situation is that there is only one trigger button provided on the handheld component, where the trigger button can be used to trigger the control module to switch modes, however, it should be noted that the trigger button itself has a press/release operation corresponding to the control energy output/interruption, therefore, it is necessary to adopt a method of non-clicking or non-long pressing the trigger button, such as double clicking or multi clicking the trigger button within a predefined short time;
The second situation is that there are multiple trigger buttons provided on the handheld component, or as shown in Figure 20, in addition to the trigger button 14, there are other operation buttons 15, such as a function key (used to select the cutting function or condensation function), a power shift button (used to adjust the power), operation buttons configured to control the rotation, bending, and opening and closing of a function head, and so on, and the control module can be triggered to activate the foot mode using a single operation button or a combination of multiple operation buttons other than the trigger button on the handle, such as double clicking (multi clicking) the function key or power shift button, or simultaneously pressing two or three of the function key, the power shift button and the trigger button, or according to a predefined pressing order of the function key - power shift button - trigger button or pressing orders in other free combinations (not limiting a certain button to single sorting and usage);
The third situation is that a new button designed specifically for this mode switching can be used to achieve trigger mode switching, such as a mode switching button similar to the function key, or a dial switch that switches to the first working mode when flipped to a first position, or to the second working mode when flipped to a second position.

In an embodiment of the present disclosure, an energy instrument for surgical use is provided, which has a first working mode and a second working mode, and the embodiment of the present disclosure can be applied in the field of ultrasonic scalpels, such as soft tissue cutting and coagulation scalpels and ultrasonic osteotomes; it can also be applied in the field of laser knives, electric knives, or other surgical scalpels.

The energy instrument for surgical use comprises an energy generating apparatus, a handheld component, and a control module, which are described as follows:
The energy generating apparatus is configured to generate energy, and if it corresponds to the application scenario of the ultrasonic scalpel, the energy generating apparatus is an ultrasonic generator; if it corresponds to the application scenario of the laser knife, the energy generation apparatus is a laser generator; if it corresponds to the application scenario of the electric knife, the energy generation apparatus is an electrical signal generator.

The present disclosure is not limited to the three types of surgical energy instruments mentioned above, but can also be used for other medical instruments that can be connected to foot switches, for example, if the energy instrument for surgical use is a medical smoking device, the energy generating apparatus is an air pump, and the medical smoking device is used in the operating room to keep the surgical environment in a smoke-free state;
the energy instrument for surgical use is a vacuum suction apparatus, and the energy generating apparatus is a vacuum pump;
the energy instrument for surgical use is an infusion pump, and the energy generating apparatus is an infusion pump;
the energy instrument for surgical use is a manipulator with an elbow joint rotation, and the energy generating apparatus is a motor;
the energy instrument for surgical use is a monopolar electrocoagulation pencil or bipolar electrocoagulation forceps, and the energy generating apparatus is an electrical signal generator, and so on, which will not enumerate here. There are two existing buttons for the above products, namely a high-power button and a low-power button, the original modes are to close the circuit when being pressed and open the circuit when being released; using the scheme of this embodiment to switch the working mode, for example, pressing and holding the high-power and low-power buttons simultaneously for 2 seconds (this method is only an example) can switch to the foot mode, and pressing the high-power button again will continue to output high power, and the high power output will not stop until the high-power button is pressed again; the same applies to the low-power button.

The handheld component is electrically connected to the energy generating apparatus via an electronic controllable switch, the handheld component is provided with one or more operation buttons, wherein at least one of the operation buttons is the trigger button 14; taking ultrasonic scalpels as an example, different models of ultrasound scalpels are provided with different operation buttons on the handles thereof, for example, some are provided with one trigger button 14, while others are provided with corresponding trigger buttons 14 for different functions (such as cutting or coagulation) or different powers, in addition to the trigger button 14, there may also be other operation buttons 15, such as a function button (used to select cutting or coagulation functions), a power shift button (used to adjust the power), etc.. The present disclosure does not limit the type of buttons on the handheld component, except for the trigger button 14, or whether there are other operation buttons 15 other than the trigger button 14, and moreover, the present disclosure does not limit the arrangement relationship between the energy generation apparatus and the handheld component to an integrated arrangement manner or the arrangement mode of the energy generation apparatus being inside the handheld component.

An input terminal of the control module is electrically connected to the operation buttons, and an output terminal thereof is electrically connected to the electronic controllable switch; the control module controls the energy instrument for surgical use to switch between the first working mode and the second working mode according to a predefined first trigger signal and a predefined second trigger signal, wherein the first trigger signal and the second trigger signal may be the same action or may each be defined as different actions, and below, the first working mode and the second working mode are first explained, and then how to switch between the two different working modes is explained.

The two working modes are shown in Figure 21:
The first working mode is the normal mode (hand-controlled mode), and the control logic for a single trigger button of the energy instrument for surgical use standby in the normal mode is: if the trigger button is pressed, the control module controls the electronic controllable switch to switch on, at this moment the instrument closes the circuit, and the energy generating apparatus continuously outputs energy to the handheld component; if the trigger button is released, the control module controls the electronic controllable switch to switch off, at this moment the instrument cuts off the circuit, at this moment the energy generating apparatus stops outputting energy to the handheld component, and returns to the standby mode. That is, under the premise of powering on the system, only during the time period when the trigger button is pressed, the energy instrument for surgical use is in working mode, and other time periods are in standby mode.

The second working mode is the "foot mode", and the control logic for a single trigger button of the energy instrument for surgical use standby in the foot mode is: a single press of the trigger button will change the state of the energy instrument for surgical use before and after the press, taking the energy instrument for surgical use being currently in standby mode (the electronic controllable switch is off) as an example, if the trigger button is pressed, the control module controls the electronic controllable switch to switch on, at this moment the instrument closes the circuit, the energy generating apparatus continuously outputs energy to the handheld component, even if the trigger button is released, the energy output is maintained continuously; the control module will not control the electronic controllable switch to switch off until the trigger button is pressed again, at this moment the instrument cuts off the circuit, at this moment the energy generating apparatus stops outputting energy to the handheld component, and returns to the standby mode.

It should be noted that the definition of the second working mode as the "foot mode" refers to this new control mode that can reduce the requirements for force and coordination on the hands of surgeon without the need for a foot switch, achieving a stable control level that is basically the same as using a foot switch, rather than referring to the mode achieved by using a foot switch. However, the present disclosure does not exclude the possibility of using a foot switch, and only means that finger coordination without the use of a foot switch can be basically achieved or even exceeded that when using a foot switch.

Below is an explanation of how to switch between two different working modes in various different embodiments:
In one embodiment of the present disclosure, one or more of the operation buttons are configured to act in accordance with a predefined operation to trigger the control module, including but not limited to the following trigger methods:
clicking or long pressing one of the operation buttons other than the trigger button; double or multiple clicking one or more of the operation buttons;
pressing multiple operation buttons simultaneously;
pressing multiple operation buttons successively according to a predefined order;
flipping a dial switch in the operation buttons.

In summary, the trigger button can be used to trigger the control module to switch modes, however, it should be noted that the trigger button itself has a press/release operation corresponding to the control energy output/interruption, therefore, it is necessary to adopt a method of non-clicking or non-long pressing the trigger button, such as double clicking or multi clicking the trigger button within a predefined short time; the control module can also be triggered to activate the foot mode using a single operation button or a combination of multiple operation buttons other than the trigger button on the handle, such as double clicking (multi clicking) the function key or power shift button, or simultaneously pressing two or three of the function key, the power shift button and the trigger button, or according to a predefined pressing order of the function key - power shift button - trigger button or pressing orders in other free combinations (not limiting a certain button to single sorting and usage); a new button designed specifically for this mode switching can also be used to achieve trigger mode switching, such as a mode switching button similar to the function key, or a dial switch that switches to the first working mode when flipped to a first position, or to the second working mode when flipped to a second position.

In one embodiment of the present disclosure, the control module is connected to an external human-machine interaction device through a wired or wireless mode, and an instruction is input to the control module through the human-machine interaction device to trigger the control module. For example, a manner that the control module is in wired connection to the touch screen, and through the operation on the touch screen, the corresponding normal mode or foot mode can be controlled and activated; an application program (App) on a mobile terminal that is wirelessly connected to the control module can also be chosen and used to switch between the normal mode and the foot mode.

The above implementations through the operation buttons and the human-machine interaction device can be independently implemented, but it is not ruled out that the above different implementation schemes can be integrated into the system of the same energy instrument for surgical use.

In one embodiment of the present disclosure, after being powered on, the energy instrument for surgical use is initialized to the first working mode, when the first trigger signal triggers the control module, the control module controls the system to switch to the second working mode, which activates the foot mode; when an external canceling trigger action is received, the control module controls the system to switch to the first working mode, namely returns to the normal mode. Obviously, the initialization state can also be set to the second working mode, and a preset operation action of the operation button for switching from the first working mode to the second working mode can be the same or different from a preset operation action of the operation button for switching the system from the second working mode to the first working mode.

The present disclosure provides an energy instrument for surgical use which can switch the working modes, which takes into account the traditional working mode that when the trigger button on the handle of the energy instrument for surgical use is pressed, the instrument outputs energy, and if the trigger button is released, the instrument stops outputting energy, and which, without the need for a foot switch, provides a working mode of foot mode that changes the control mode of the trigger button on energy output, the control method (mode switching operation) comprises the following steps:
in the case that the energy instrument for surgical use is powered on, inputting a predefined trigger action to redefine a control mode of the trigger button on energy output to: no energy output in an initial state, and if the trigger button is pressed for the first time, the instrument outputs energy, and the instrument will not stop outputting energy until the trigger button is pressed again;
the trigger button will not restore the initial control mode on energy output until the energy instrument for surgical use is disconnected from the power supply or a predefined trigger action is input.

The embodiment of this control method belongs to the same concept as the above embodiments of the energy instruments for surgical use, and the above embodiments of the energy instruments for surgical use are incorporated herein into this manipulation method embodiment by reference in their entireties.

The traditional working mode of the trigger button on the energy instruments for surgical use is to press to close the circuit, and release to open the circuit, and this traditional normal mode is already in line with the usage habits of surgeons, especially since the surgical ability of surgeons is gradually accumulated based on previous surgical experience, this traditional design cannot be canceled; secondly, from an ergonomic perspective, the normal mode (hand-controlled mode) is a relatively safe design, and if the blade of the instrument accidentally touches human tissue that is not a surgical target during energy exciting, it may cause unnecessary damage.

In the traditional normal mode that needs to be preserved, during the stage of continuous energy output in an operation, the scheme of this embodiment can be used to switch the working mode of the trigger button through external trigger switching operation, so that the surgical practitioner does not need to keep pressing the trigger button with their fingers, nor does they need to keep stepping on the foot switch, which avoids damage or pain to the finger bones caused by the surgeon's constant pressing of the trigger button during a prolonged operation period, and avoids the leg burden caused by the surgeon's single foot (the foot that has not pressed the foot switch) supporting the body weight for a long time, and which at the same time can achieve finger coordination that is basically consistent with when using the foot switch.

It should be noted that herein, relational terms such as first and second are only used to distinguish one entity or operation from another entity or operation, and do not necessarily require or imply any actual relationship or order between these entities or operations. Moreover, terms "comprising", "including", or any other variation thereof are intended to encompass non-exclusive inclusion, such that a process, method, item, or device that includes a series of elements not only includes those elements, but also includes other elements that are not explicitly listed, or also include elements inherent in such a process, method, item, or device. Without further limitations, elements limited by the statement "comprising a..." do not exclude the existence of other identical elements in the process, method, item, or device that includes the elements.

The above are only specific implementations of the present application. It should be noted that, for those ordinary skilled/of ordinary skill in the art, any improvements or modifications can be made without depart from the technical principle and conception of the present application, and shall be covered by the protective scope of the present application.

## Claims

1. An ultrasonic scalpel comprising a handheld component and a control module, the handheld component being provided with a trigger button, **characterized in that**, the trigger button is connected to an input terminal of the control module;
the control module is configured to be electrically connected to an energy generating apparatus, if the trigger button is pressed, the control module controls the energy generating apparatus to output ultrasonic energy, and if the trigger button is released, the control module controls the energy generating apparatus to stop outputting ultrasonic energy;
the control module is configured to execute the following procedure:
in response to a first trigger signal triggered by an external switching action, each time the trigger button is pressed, the control module controls the energy generating apparatus to change the current ultrasonic energy output state, and the changed ultrasonic energy output state is maintained until the trigger button is pressed again.

2. The ultrasonic scalpel according to claim 1, **characterized in that**, the control module is configured to be electrically connected to the energy generating apparatus via a power board, if the trigger button is pressed, the control module controls the power board to send a drive instruction to the energy generating apparatus, and if the trigger button is released, the control module controls the power board to stop sending the drive instruction;
the control module is configured to execute the following procedure:
in response to the first trigger signal, each time the trigger button is pressed, the control module controls the power board to change the current drive instruction output state, and the changed drive instruction output state is maintained until the trigger button is pressed again, wherein, the drive instruction output state is divided into a state of sending the drive instruction and a state of stopping sending the drive instruction.

3. The ultrasonic scalpel according to claim 1, **characterized in that**, the control module is configured to be electrically connected to the energy generating apparatus via an electronic controllable switch, if the trigger button is pressed, the control module controls the electronic controllable switch to switch on, and if the trigger button is released, the control module controls the electronic controllable switch to switch off;
the control module is configured to execute the following procedure:
in response to the first trigger signal, each time the trigger button is pressed, the control module controls the electronic controllable switch to change the current switching state, and the changed switching state is maintained until the trigger button is pressed again.

4. The ultrasonic scalpel according to any one of claims 1 to 3, **characterized in that**, the control module is configured to execute the following procedure:
after responding to the first trigger signal, in response to a second trigger signal triggered by an external release switching action, if the trigger button is pressed, the control module controls the energy generating apparatus to output ultrasonic energy, and if the trigger button is released, the control module controls the energy generating apparatus to stop outputting ultrasonic energy;
wherein, the external release switching action is the same as or different from the external switching action.

5. The ultrasonic scalpel according to any one of claims 1-3, **characterized in that**, the external switching action that triggers the first trigger signal comprises:
performing a predefined operation action on the trigger button, comprising double or multiple clicking the trigger button within a predefined time period.

6. The ultrasonic scalpel according to any one of claims 1-3, **characterized in that**, the trigger button is arranged on a housing of the handheld component, there is one or more trigger buttons, and the housing of the handheld component is provided with other operation buttons thereon, and the other operation buttons comprise function keys and/or power shift buttons and/or operation buttons configured to control any of rotation, bending, opening and closing operations of a functional head.

7. The ultrasonic scalpel according to claim 6, **characterized in that**, the external switching action that triggers the first trigger signal comprises: performing the following operation on one or more of the trigger button and the other operation buttons:
clicking or long pressing one of the operation buttons other than the trigger button; or,
double or multiple clicking one or more of the operation buttons within a predefined time period; or,
pressing multiple operation buttons simultaneously; or,
pressing multiple operation buttons successively according to a predefined order; or,
flipping a dial switch in the operation buttons.

8. The ultrasonic scalpel according to any one of claims 1-3, **characterized in that**, no joint or interface is provided on the handheld component to connect to a foot switch.

9. The ultrasonic scalpel according to any one of claims 1-3, **characterized in that**, the handheld component comprises a handle shell, a transducer assembly and a power cord, wherein, the control module and the transducer assembly are arranged within an accommodating cavity of the handle shell, and the transducer assembly is rotatably arranged around an axis in the accommodating cavity;
one end portion of the power cord is connected to the transducer assembly in the accommodating cavity, and the other end portion of the power cord goes through a lower portion of the handle shell to the outside of the accommodating cavity.

10. The ultrasonic scalpel according to claim 9, **characterized in that**, the transducer assembly comprises a transducer housing and a transducer that are fixedly arranged to each other, the transducer housing has a hollow cavity, at least a rear portion of the transducer is accommodated within the hollow cavity, and the transducer assembly further comprises a conductive element fixedly arranged outside the transducer housing;
the conductive element has at least a conductive portion, the transducer is fixedly and electrically connected to the conductive portion, the accommodating cavity of the handle shell is further fixedly provided with a power connection element therein, the power connection element abuts against the conductive portion, and in the process that the transducer assembly rotates about its own axis with respect to the handle shell, the power connection element is always in contact with the conductive portion to maintain electric connection.

11. The ultrasonic scalpel according to claim 10, **characterized in that**, the conductive element is fixedly arranged at an outer peripheral portion of the rear en portion of the transducer housing, the conductive portion is in a shape of a circular ring, and the axis of the conductive portion is collinear with the axis of the transducer assembly, the power connection element is located on the circumferential outer side of the transducer assembly, and the power connection element elastically abuts against the conductive portion forwards along the front-rear direction of the transducer housing.

12. The ultrasonic scalpel according to any one of claims 1 to 3, **characterized in that**, the external switching action that triggers the first trigger signal in response to the first trigger signal comprises:
the control module is connected to an external human-machine interaction device through a wired or wireless mode, and an instruction is input to the control module through the human-machine interaction device.

13. The ultrasonic scalpel according to claim 12, **characterized in that**, the human-machine interaction device is a touch screen, and the touch screen is in wired connection to the control module; or, the human-machine interaction device is a mobile terminal, and the mobile terminal is wirelessly connected to the control module.

14. An energy instrument for surgical use, **characterized in that**, it has a first working mode and a second working mode, and the energy instrument for surgical use comprises:
an energy generating apparatus configured to generate energy;
a handheld component electrically connected to the energy generating apparatus via an electronic controllable switch, the handheld component being provided with one or more operation buttons, wherein at least one of the operation buttons is a trigger button;
a control module with an input terminal electrically connected to the operation buttons, and an output terminal electrically connected to the electronic controllable switch; the control module being configured to control the energy instrument for surgical use to switch from the first working mode to the second working mode according to a first trigger signal triggered by an external switching action, and to switch from the second working mode to the first working mode according to a second trigger signal triggered by an external release switching action, the external release switching action being the same as or different from the external switching action;
when the energy instrument for surgical use is in the first working mode, if the trigger button is pressed, the control module controls the energy generating apparatus to output ultrasonic energy, and if the trigger button is released, the control module controls the energy generating apparatus to stop outputting ultrasonic energy;
when the energy instrument for surgical use is in the second working mode, in response to that each time the trigger button is pressed, the control module controls the energy generating apparatus to change the current energy output state, and the changed energy output state is maintained until the trigger button is pressed again.

15. The energy instrument for surgical use according to claim 14, **characterized in that**, the control module is configured to be electrically connected to the energy generating apparatus via a power board, if the trigger button is pressed, the control module controls the power board to send a drive instruction to the energy generating apparatus, and if the trigger button is released, the control module controls the power board to stop sending the drive instruction;
the control module is configured to execute the following procedure:
in response to the first trigger signal, each time the trigger button is pressed, the control module controls the power board to change the current drive instruction output state, and the changed drive instruction output state is maintained until the trigger button is pressed again, wherein, the drive instruction output state is divided into a state of sending the drive instruction and a state of stopping sending the drive instruction.

16. The energy instrument for surgical use according to claim 14, **characterized in that**, the control module is configured to be electrically connected to the energy generating apparatus via an electronic controllable switch, if the trigger button is pressed, the control module controls the electronic controllable switch to switch on, and if the trigger button is released, the control module controls the electronic controllable switch to switch off;
the control module is configured to execute the following procedure:
in response to the first trigger signal, each time the trigger button is pressed, the control module controls the electronic controllable switch to change the current switching state, and the changed switching state is maintained until the trigger button is pressed again.

17. The energy instrument for surgical use according to any one of claims 14 to 16, **characterized in that**, the control module controlling the instrument to switch the working mode according to the first trigger signal and the second trigger signal comprises:
one or more of the operation buttons are configured to act in accordance with a predefined operation to trigger the control module.

18. The energy instrument for surgical use according to claim 17, **characterized in that**, one or more of the operation buttons are configured to trigger the control module according to the following operation mode:
clicking or long pressing one of the operation buttons other than the trigger button; or,
double or multiple clicking one or more of the operation buttons; or,
pressing multiple operation buttons simultaneously; or,
pressing multiple operation buttons successively according to a predefined order; or,
flipping a dial switch in the operation buttons.

19. The energy instrument for surgical use according to any one of claims 14 to 16, **characterized in that**, the operation buttons comprise a trigger button and other operation buttons, and the other operation buttons comprise function keys and/or power shift buttons and/or operation buttons configured to control any of rotation, bending, opening and closing operations of a functional head.

20. The energy instrument for surgical use according to any one of claims 14 to 16, **characterized in that**, the control module controlling the instrument to switch the working mode according to the first trigger signal and the second trigger signal comprises:
the control module is connected to an external human-machine interaction device through a wired or wireless mode, and an instruction is input to the control module through the human-machine interaction device to trigger the control module.

21. The energy instrument for surgical use according to claim 20, **characterized in that**, the human-machine interaction device is a touch screen, and the touch screen is in wired connection to the control module; or,
the human-machine interaction device is a mobile terminal, and the mobile terminal is wirelessly connected to the control module.

22. The energy instrument for surgical use according to any one of claims 14 to 16, **characterized in that**, after being powered on, the energy instrument for surgical use is initialized to the first working mode, or
after being powered on, the energy instrument for surgical use is initialized to the second working mode.

23. The energy instrument for surgical use according to any one of claims 14 to 16, **characterized in that**, the energy instrument for surgical use is an ultrasonic scalpel, the energy generating apparatus is an ultrasonic generator, and the ultrasonic generator is arranged inside or outside the handheld component; or,
the energy instrument for surgical use is a laser knife, the energy generating apparatus is a laser generator, and the laser generator is arranged inside or outside the handheld component; or,
the energy instrument for surgical use is an electric knife, the energy generating apparatus is an electrical signal generator, and the electrical signal generator is arranged inside or outside the handheld component; or,
the energy instrument for surgical use is a medical smoking device, and the energy generating apparatus is an air pump; or,
the energy instrument for surgical use is a vacuum suction apparatus, and the energy generating apparatus is a vacuum pump; or,
the energy instrument for surgical use is an infusion pump, and the energy generating apparatus is an infusion pump; or,
the energy instrument for surgical use is a manipulator with an elbow joint rotation, and the energy generating apparatus is a motor; or,
the energy instrument for surgical use is a monopolar electrocoagulation pencil or bipolar electrocoagulation forceps, and the energy generating apparatus is an electrical signal generator.

24. A control method for an energy instrument for surgical use, the controlled energy instrument for surgical use comprising a trigger button, an initial control mode of the trigger button on energy output being: if the trigger button is pressed, the instrument outputs energy, and if the trigger button is released, the instrument stops outputting energy, **characterized in that**, changes the control mode of the trigger button on energy output without a foot switch, and the control method comprises the following steps:
in the case that the energy instrument for surgical use is powered on, inputting a predefined trigger action to redefine a control mode of the trigger button on energy output to: no energy output in an initial state, and if the trigger button is pressed for the first time, the instrument outputs energy, and the instrument will not stop outputting energy until the trigger button is pressed again;
the trigger button will not restore the initial control mode on energy output until the energy instrument for surgical use is disconnected from the power supply or a predefined trigger action is input.

25. The control method according to claim 24, **characterized in that**, the control mode is applied to the following energy instrument for surgical use:
the energy instrument for surgical use is an ultrasonic scalpel, the energy generating apparatus is an ultrasonic generator, and the ultrasonic generator is arranged inside or outside the handheld component; or,
the energy instrument for surgical use is a laser knife, the energy generating apparatus is a laser generator, and the laser generator is arranged inside or outside the handheld component; or,
the energy instrument for surgical use is an electric knife, the energy generating apparatus is an electrical signal generator, and the electrical signal generator is arranged inside or outside the handheld component; or,
the energy instrument for surgical use is a medical smoking device, and the energy generating apparatus is an air pump; or,
the energy instrument for surgical use is a vacuum suction apparatus, and the energy generating apparatus is a vacuum pump; or,
the energy instrument for surgical use is an infusion pump, and the energy generating apparatus is an infusion pump; or,
the energy instrument for surgical use is a manipulator with an elbow joint rotation, and the energy generating apparatus is a motor; or,
the energy instrument for surgical use is a monopolar electrocoagulation pencil or bipolar electrocoagulation forceps, and the energy generating apparatus is an electrical signal generator.
